Europäisches Patentamt

European Patent Office

Office européen des brevets

(19)

(11) Publication number : 0 494 054 A2

## (12) EUROPEAN PATENT APPLICATION

(21) Application number : 91830580.6

(22) Date of filing : 20.12.91

(51) Int. Cl.⁵ : A61N 5/04

(30) Priority : 31.12.90 IT 955990

(43) Date of publication of application :
08.07.92 Bulletin 92/28

(84) Designated Contracting States :
AT BE CH DE DK ES FR GB GR LI LU MC NL SE

(71) Applicant : CONSIGLIO NAZIONALE DELLE
RICERCHE
Piazzale Aldo Moro, 7
I-00185 Roma (IT)

(72) Inventor : Andreuccetti, Daniele
Via E. Nencioni n.6
I-50135 Firenze (IT)
Inventor : Bini, Marco
Via Monte Sabotino n.39
I-51100 Pistoia (IT)

Inventor : Ignesti, Amleto
Via R. Sernesi n.7
I-50142 Firenze (IT)
Inventor : Olmi, Roberto
Via Senese n.245/D
I-50124 Firenze (IT)
Inventor : Priori, Saverio
Via delle Prata n.8
I-50010 Scandicci, Firenze (IT)
Inventor : Rubino, Nicola
Via de' Bardi n.36
I-50125 Firenze (IT)
Inventor : Vanni, Riccardo
Isolato Boccaccio interno H/2
I-50018 Scandicci, Firenze (IT)

(74) Representative : Mannucci, Michele
Ufficio Tecnico Ing.A. Mannucci, Via della
Scala 4
I-50123 Firenze (IT)

(54) Compact emission antennas on high-permittivity ceramic for use in electromagnetic hyperthermia.

(57) Emission antenna for hyperthermia having optimised physical dimensions and which heats the biological tissues in depth and uniformly, with operating frequencies below 500 MHz, and focusing the RF energy (phased array). The use of a dielectric ceramic of high permittivity ($\varepsilon_r$ = 80 ; 160), in conjunction with the technology of microstrips (patch antennas), permits the construction of emission antennas of extremely restricted dimensions and of reduced weight, having operating frequencies below 500 MHZ. The ceramic employed has low losses and permits very high levels of efficiency to be achieved. The emission antenna may be used on its own or may be employed advantageously in the construction of emission antennas which are flexible and conformable, in order to obtain a uniformity of deposition of power exceeding that which can be achieved using conventional emission antennas, with the same surface area and frequency. It is particularly suitable as a module for the construction of phased arrays.

Fig.1

EP 0 494 054 A2

The object of the invention is to construct improved emission antennas for hyperthermia, in order to obtain effective action with appropriately restricted physical dimensions, which emission antennas are capable of heating the biological tissues in depth and uniformly. The basis for the invention is constituted by the adoption of the technology of microstrip antennas (patch antennas) in conjunction with the use of an interposed solid dielectric, which consists of a ceramic with a high dielectric constant and low losses.

This solution permits the construction of emission antennas having an operating frequency below 500 MHz and of reduced physical dimensions, which can expediently be assembled in order to achieve the object of a concentration of electromagnetic energy in depth in the biological tissue (phased array).

An earlier patent held by the same proprietor (Italian Patent No. 1,198,876 filed on 27 June 1984, Application No. 9449 A/84) relates to an emission antenna for hyperthermia, which emission antenna is suitable for the heat treatment of biological tissues of limited depth, and comprises components in a waveguide, said components forming a cavity filled with water, which cavity is further delimited by a nonmetallic wall, which is flexible and deformable and which is capable of being supported on and adapting to the tissue.

Microstrip antennas (patch antennas) have also been devised, which make use of water as dielectric. The experience gained using these has demonstrated certain positive features of this type of emission antenna, including the following: reduced physical dimensions; good adaptation to the biological tissues to be treated; incorporated bolus; ease of construction; good distribution of the RF power in the biological tissue simulators.

However, there are certain disadvantages in microstrip emission antennas: the use of deionised water is not sufficient to reduce to a negligible level the energy losses in the emission antenna, such losses causing a heating of the liquid interposed between the two metal planes of the microstrip (in particular on the open edge), as a result of the strong electric fields present there. This heating gives rise to a significant shift (250 kHz/°C at 100 mHz) in the resonant frequency of the antenna during operation and makes it essential to use very complex power generators, which are equipped with devices for the automatic tracking of the frequency. This furthermore reduces the efficiency of the emission antenna [efficiency = (power transferred to the tissue)/(power generated)], and increases the load of the cooling circuit which has to remove this additional energy.

According to the invention, with the object of eliminating the disadvantages enumerated hereinabove, there has been provided the use, in the emission antenna incorporating a microstrip antenna, of a solid dielectric having a dielectric constant similar to that of water but with low losses. A particularly suitable material is a ceramic material (AT-D-80), which has been produced, to order, using typical techniques by the Ampex Corporation, Sunnyvale,.California (USA), in order to obtain the specificelectrical characteristics aimed at, and in respect of which the development has taken place of technologies of mechanical working, chemical treatments and electronic technologies in accordance with particular features of the present invention for the purpose of constructing the microstrip hyperthermia emission antenna in question.

Further features of the invention are defined in the subclaims.

The use of a dielectric ceramic of high permittivity ($\varepsilon_r$ = 80; 160), in conjunction with the technology of microstrips (patch antennas), permits the construction of emission antennas of extremely restricted dimensions and of reduced weight, having operating frequencies below 500 MHz. The ceramic employed offers low losses and permits very high levels of efficiency to be achieved.

Among the principal features of this type of radiator, mention may be made of the following: absence of reactive fields in the tissue being treated; high efficiency of conversion; insensitivity of the operating frequency to temperature (temperature stability); a broad frequency band (tens of MHz); retention of all the positive features of patch emission antennas which use deionised water as dielectric, i.e. reduced physical dimensions, good adaptation to the biological tissue, incorporated bolus, ease of construction, good distribution of the RF power (experimentally assessed in biological tissue simulators).

The emission antenna in question may be used on its own or may advantageously be employed in the construction of flexible and conformable emission antennas, in order to obtain a uniformity of deposition of power exceeding that which can be achieved using conventional emission antennas, with the same surface area and frequency. It is particularly suitable for use as a module for the construction of phased arrays.

A particular effectiveness is achieved by adopting a quarter wave microstrip radiator module, designed for 434 MHz.

The accompanying drawing permits a better understanding of particular embodiments which are described hereinbelow. In the drawing:

Fig. 1 shows a microstrip radiator module, in a diagrammatic perspective view;

Fig. 2 shows an electrical circuit diagram for the assessment of the resonance length;

Figs. 3 and 4 show, in cross section and in a perspective view in the direction of the arrow flV, a radiator according to the invention;

Fig. 5 shows an emission antenna assembly incorporating the radiator referred to hereinabove and the bolus.

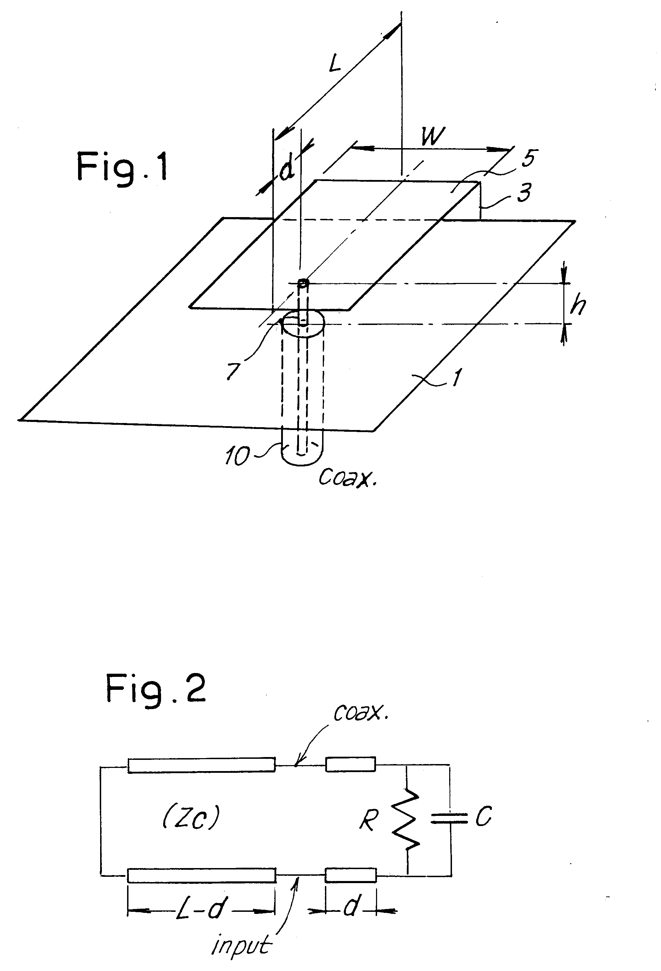

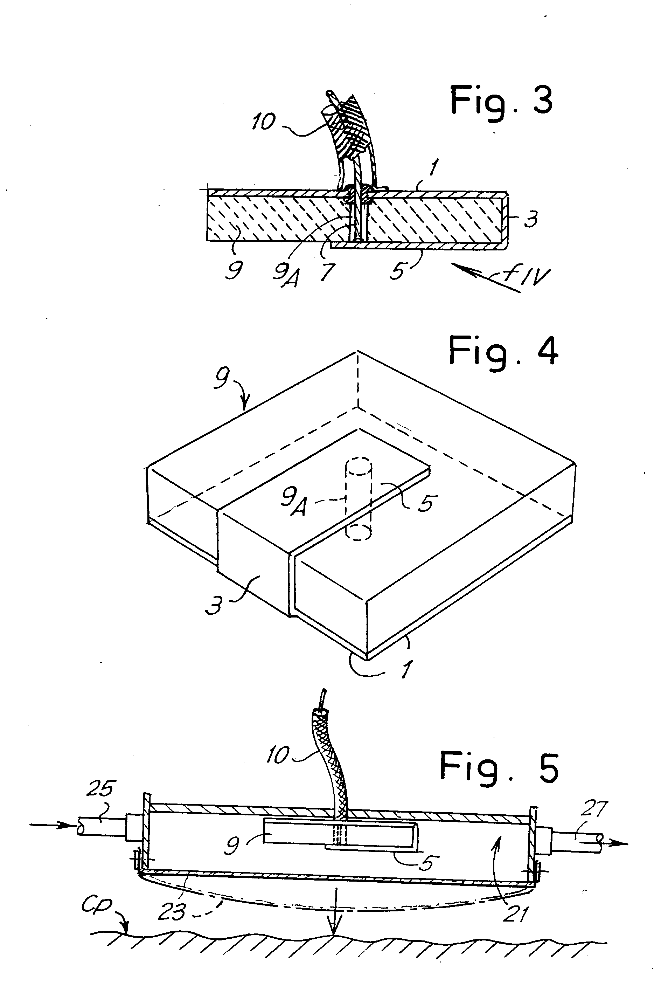

With reference to Fig. 1, an emitter or radiator has a ground plane 1, a connection 3 and a microstrip 5. $\underline{h}$ indicates the distance between the parts 1 and 5; W and L indicate the dimensions of the microstrip 5; $\underline{d}$ indicates the distance of the exciter 7 from the edge of the microstrip 5. 10 indicates a coaxial supply cable. The dielectric between the parts 1 and 5 is formed by the particular dielectric ceramic 9 in the form of a slab of thickness $\underline{h}$, which is pierced at 9A (see Fig. 4) for the passage of the exciter 7.

In practice, the laminar parts 1, 3 and 5 are obtained by metallising the surfaces of the slab 9.

The radiator module is formed by a segment of open line on one side (quarter wave resonant patch) or on both sides (half wave patch).

The theoretical analysis of the quarter wave microstrip radiator module described hereinabove is based on the following model: the antenna is seen as an open resonator, consisting of a segment of microstrip transmission line which is short circuited at one end and open at the other, as indicated in Fig. 1.

The energy emitted from the open end propagates in a direction perpendicular to the surface of the patch 5.

With reference to the model referred to hereinabove, the following limits are identified for the quantities to be fixed or to be computed in the design phase of the emission antenna:

– width W of the patch: maximum limit of $\lambda$ or $\lambda/2$;

– length L of the patch: approximately $\lambda/4$ or $\lambda/2$; a more specific statement on this matter is given hereinbelow;

– distance $\underline{h}$ of the patch 5 from the ground plane 1: maximum limit of $\lambda/10$ corresponding to a Q of 2.5;

– dimensions of the ground plane, minimum: (W+4h)x(L+2h).

The parameter which requires the most accurate computation is the length L of the radiator element (patch) 5, as this determines the resonant frequency of the structure. The resonant length L is assessed by means of the model shown in Fig. 2, which represents a segment of microstrip line which is short circuited at one end and closed onto-its fringing capacitance from the open end.

In the model according to Figs. 1 and 2, the conductance G takes into account the energy losses due to irradiation. The capacitance C of the open end is computed by means of semi-empirical formulae. Using $Z_c$ to designate the characteristic impedance of the line, $\beta$ to designate the propagation constant in the dielectric substrate and $\omega$ to designate the resonant frequency, the length L is given by:

$$L = \tan^{-1}(1/\omega \, CZ_c)/\beta$$

A list is given hereinbelow of other quantities of interest in the design phase.

Q of the resonator:

$Q = (h/L_{eq} + \tan \delta)^{-1}$ (disregarding the resistive losses)

Bandwidth (ROS$\leqq$2):

BW = 70.71/Q [%]

Input resistance at resonance ($R_{in}$):

$$Rin = \frac{(Y_c \cos\beta d - \omega C \sin d\beta d)^2 + G^2 \sin d^2\beta d}{Y_c^2 G}$$

where $\underline{d}$ is the distance from the edge of the exciter and $Y_c = 1/Z_c$ is the characteristic admittance of the line. The impedance matching to 50$\Omega$ of the generator is obtained in the standard manner by means of L, T or $\pi$ reactive circuits.

Table I hereinbelow indicates the dimensions L, W and $\underline{h}$ of some $\lambda/4$ patches constructed on a substrate of dielectric constant $\varepsilon_r = 80$:

TABLE I

| Frequency (MHz) | L (mm) | V (mm) | h (mm) |
|---|---|---|---|
| 101.5 | 75 | 100 | 5 |
| 178.8 | 40.7 | 50 | 4.3 |
| 340 | 19.5 | 30 | 3.5 |
| 434 | 14.2 | 30 | 3.3 |

Finally, Table II hereinbelow gives the principal characteristics measured on a module operating at 434 MHz.

## TABLE II

| | |
|---|---|
| Operating frequency (MHz) | 434 |
| Area of the patches (cm) | 1.42 × 3 |
| External dimensions (cm) | 3 × 5 × .33 |
| Band (ROS≤2) | 14% |
| Efficiency | 70% |
| Characterisation (ESHO): | |
| Penetration depth (cm) | 1.9 |
| Effective field size (cm) | 5.7 × 2.5 |

An emission antenna according to the invention may comprise a plurality of antennas, i.e. a plurality of emitters positioned in various ways on a mutual basis. Two supply modes are possible: each emission antenna powered by an independent generator or the antennas powered by coherent generators with phase relations which are variable in order to obtain the desired focusings in the tissue to be treated.

In Fig. 5, 21 indicates the bolus of the emission antenna, one of the walls 23 of which is flexible, for example made of rubber, for application by adhesion to the epithelium of the body CP in which the treatment is to be performed. The bolus 21 contains the emission antenna 1, 3, 5, 9, to which the cable 10 is connected; conduits 25 and 27 pass to the bolus 21 for the circulation of the deionised water which cools the surface of the biological tissue (CP) via the wall 23.

With regard to the nature of the dielectric, it is noted that in the catalogues of Ampex there were already in existence dielectrics for microwave resonators ATD-51 (dielectric constant 51) and ATD-100 (dielectric constant 100) with microwave loss factors tan δ < 0.0003 and with negligible variations in the dielectric constant with temperature.

These favourable characteristics have prevailed upon the inventors to seek to modify the formula of the mixture of the ATD dielectrics in such a manner as to obtain a ceramic having a dielectric constant of 80. This has lead to the construction of a novel dielectric ATD-80 in the form of parallelepipeds having dimensions 2x6x1/4 inches and 2x6x1/8 inches, with lapped surfaces, which satisfied the constructional requirements of the invention. The ceramic has the appearance of a tile of light brown colour which darkens slightly when it is exposed to light; it is a material which is very hard and fragile. One possible composition is obtained by mixing, in expedient proportions, ceramic powders containing barium and titanium oxides (in order to increase the dielectric constant,thereof) which are pressed and fired in the manner of normal tiles, above 1000°C. The physical characteristics are set forth in Table III:

## TABLE III

PHYSICAL CHARACTERISTICS, ATD-80

| | |
|---|---|
| Specific gravity | 3.81 $(g/cm^3)$ |
| Dielectric constant $\epsilon_r$ | 78.75 |
| Dielectric loss tan $\delta$ | 0.00086 |
| Temperature coefficient $\epsilon_r$ | $< - 0.001(1/°C)$ |
| Coefficient of expansion | $(11.7 \div 7.5) \times 10^{-6}(1/°C)$ |
| Reproducibility $\epsilon_r$, tan $\delta$ | ± 5% |

The ceramic material employed is relatively hard and fragile and accordingly it cannot be worked with the normal tools used for metal working.

The material has lapped surfaces which are orthogonal to one another; further mechanical working operations are the cutting of the small slab, to obtain the correct width and length dimensions, and piercing. Cutting may be carried out using diamond discs mounted on wheels or using a filament cutting device.

The holes in the ceramic are made using diamond milling cutters.

To obtain a patch emission antenna, it is necessary to metallise the small slab of ceramic, as in Fig. 4.

In addition to the surfaces of the small slab 9, it is also necessary to metallise the hole 9A, which constitutes the injector of the electromagnetic energy into the resonance structure. Techniques which have given good results are vacuum deposition and electrodeless galvanic deposition.

Summarising the aforegoing, the principal object of the invention is that of constructing an emission antenna for hyperthermia having optimised physical dimensions which heats the biological tissues in depth and uniformly, with operating frequencies below 500 MHz and focusing the RF energy (phased array).

The use of a dielectric ceramic of high permittivity ($\varepsilon_r$ = 80; 160), in conjunction with the technology of microstrips (patch antennas), permits the construction of emission antennas of extremely restricted dimensions and of reduced weight, with operating frequencies below 500 MHz. The ceramic employed has low losses and permits very high levels of efficiency to be achieved.

The principal characteristics of this type of radiator are the following:

- Absence of reactive fields in the tissue being treated;
- High efficiency of conversion;
- Insensitivity of the operating frequency to temperature (temperature stability);
- Broad band;
- Retention of all the positive features of patch emission antennas which use deionised water as dielectric;
- Reduced physical dimensions;
- Good adaptation to the biological tissue;
- Incorporated bolus;
- Ease of construction
- Good distribution of the RF power (assessed in biological tissue simulators).

The emission antenna may be used on its own or may be employed advantageously in the construction of emission antennas which are flexible and conformable, in order to obtain a uniformity of deposition of power exceeding that which can be achieved using conventional emission antennas, with the same surface area and frequency. It is particularly suitable as a module for the construction of phased arrays.

It is understood that the drawing shows only an exemplification which is given only by way of practical demonstration of the innovation, it being possible for this innovation to vary in terms of forms and arrangements without thereby departing from the scope of the concept which forms said innovation.

## Claims

1. A microstrip emission antenna for electromagnetic hyperthermia in organic tissues, for therapeutic purposes, said emission antenna being characterised in that it comprises a solid material of high dielectric constant and low losses, which material is pierced to receive the exciter.

2. Emission antenna according to the preceding claim, characterised in that the solid dielectric is a ceramic material, having a dielectric constant of the order of $\varepsilon_r$ = 80 or above.

3. Emission antenna according to the preceding claims, characterised in that it makes use of a different solid or semisolid material having similar electrical characteristics.

4. Emission antenna according to the preceding claims, characterised in that it is designed for a frequency within the range between 10 and 500 MHz.

5. Emission antenna according to Claim 4, characterised in that it is designed for a frequency of the order of 434 MHz.

6. Emission antenna according to the preceding claims, which is constructed by metallisation of zones of external surfaces of the solid dielectric.

7. Emission antenna according to the preceding claims, which is constructed using technologies different from those which are described hereinabove merely by way of example.

8. Emission antenna according to the preceding claims, having the following dimensions (using the symbols of Fig. 1):
   - width W of the patch: less than $\lambda$ or $\lambda/2$
   - length L of the patch: approximately $\lambda/4$ or $\lambda/2$
   - distance $\underline{h}$ between patch and ground (thickness of the dielectric: less than or equal to $\lambda/10$

– dimensions of the ground plane: at least (W+4h)x(L+2h).

9. Emission antenna according to the preceding claims, having a plurality of antennas of variable positioning.

10. Emission antenna according to the preceding claims, incorporating incoherent generators for their power supply.

11. Emission antenna according to the preceding claims, incorporating antennas powered by coherent generators with phase relations which are variable in order to achieve focusing within the tissue to be treated.

Fig.1

Fig.2

7

# Fig. 3

# Fig. 4

# Fig. 5